# EUROPEAN PATENT APPLICATION

(11) **EP 2 594 830 A1**
(43) Date of publication of application: **22.05.2013**
(21) Application number: 11189644.5
(22) Date of filing: 17.11.2011
(51) Int. Cl.: F16K 7/02, F16K 31/06, F16K 1/12

(54) **An expiratory valve for controlling a flow**

(71) Applicant: Mindray Medical Sweden AB, 174 57 Sundbyberg (SE)
(72) Inventor: Cewers, Göran, 216 42 Limhamn (SE); Ringdén, Ola, 172 37 Sundbyberg (SE); Werner, Johan, 142 52 Skogås (SE)
(74) Representative: KIPA AB

(57) **Abstract**

A valve (3) comprising a hollow flexible body (39) having a waist area (32) positioned between two fixed end points (33a,33b), flow channel located inside the hollow flexible body (39) for passage of a fluid, valve seat (30) centrally positioned in the flow channel, an annular element (31) circumferentially arranged around said hollow flexible body (39), wherein the annular element (31) is arranged to be movable towards or away from the valve seat (30) in an axial direction along the flow channel by an actuator unit, and whereby the waist area is at least partially repositioned and/or reshaped to control a flow of the fluid through the flow channel.

## Description

### BACKGROUND OF THE INVENTION

### Related applications

The present application relates to the following application of the same inventor as the present application with the following title: "VALVE AND METHOD TO CONTROL A FLOW" (PCT/EP2011/057810); which all are incorporated herein by reference in their entirety for all purposes

### Field of the Invention

The invention pertains in general to the field of valves. More particularly, the invention relates to a valve device for mechanically controlling the flow of at least one fluid through at least one channel. Even more particularly, the invention relates in some embodiments to expiratory valves for breathing machines like medical ventilators.

### Description of the Prior Art

It is known that, when designing low pressure valves, especially in the field of gas control in medical ventilators is it of high importance that the flow channel in the valve has low flow resistance and no or little turbulence. In some applications requirements are high concerning cleaning of contaminated parts of the valve. Moreover, it is often desirable for the design to be small and light and that the actuator controlling the valve may be made small and isolated from the flow channel.

Examples of low pressure valve applications are expiration valves, patient pressure relief valves, mixer valves and flow valves in low pressure systems.

Today, the commonest design of low pressure valves comprises a circular disk lying against the end of a tube forming a valve seat. US patent 5,127,400 discloses an example of such a design. The drawbacks of such a design are the complexity of the flow channel, which causes turbulence and cleaning issues. Moreover, the entire circular disk is exposed to a pressure, while the flow only depends on the outer edge of the disk. Thus an unnecessarily strong, heavy and expensive actuator is needed to control this type of valve.

Another example is international patent application WO 2008/112332 that discloses an inline valve. The inline valve includes, a housing, a choke member in operable communication with the housing, a portion of the choke member being substantially immobile relative to the housing and a portion of the choke member being mobile relative to the housing. The inline valve further includes, an actuator in operable communication with the movable portion of the choke member, the actuator selectively causing the choke member to deform radially. However, the valve disclosed in WO 2008/112332 only uses one area that can be deformed and can therefore not be used as a valve device between two fixed end positions. Further, the valve can only choke a flow from the inside against a hard conduit. WO 2008/112332 does not disclose anything regarding how to avoid deformation and wear nor how to be able to simply clean or disassemble the valve. Thus the disclosed valve is not suitable for medical devices.

United States patent number US 5,119,861 discloses a normally closed valve for controlling flow of fluid material through a straight, non-tapered conduit has resilient elastomeric seal attached to the perimeters of two axially aligned rigid end plates. Elasticity of elastomeric seal draws end plates together while radially expanding so as to engage and seal against the inner wall of conduit. This valve may be opened by axially separating the two opposing end plates using a locally actuated displacer mechanism attached to one of the end plates, causing the elastomeric seal to radially contract and the valve to open. The issue with the design of this valve is almost the same as for WO 2008112332. Even though US 5,119,861 disclose a valve having at least two resilient members the configuration of the device makes it only usable when one end is displaceable. Thus it cannot be used in-between two fixed end-points. Since the valve is normally closed, the focus and the use of more than one resilient member are to increase the sealing effect for specific applications.

Hence, an improved valve would be advantageous and in particular an actuator controlled valves allowing for increased flexibility, an improved control of the closing and opening, cost-effectiveness, and/or fulfilling the above-mentioned criteria, of a small and light actuator controlled valve having low flow resistance and no or little turbulence, would be advantageous.

### SUMMARY OF THE INVENTION

Accordingly, embodiments of the present invention preferably seek to mitigate, alleviate or eliminate one or more deficiencies, disadvantages or issues in the art, such as the above-identified, singly or in any combination by providing a device and a method, for controlling a flow through a valve, according to the appended patent claims.

According to one aspect of the invention, a valve is provided comprising, a hollow flexible body having a waist area positioned between two fixed end points, a flow channel located inside the hollow flexible body for passage of a fluid, a valve seat centrally positioned in the flow channel, an annular element circumferentially arranged around the hollow flexible body. The annular element is arranged to be movable towards or away from the valve seat in an axial direction along said flow channel by an actuator unit, and whereby the waist area is at least partially repositioned and/or reshaped to control a flow of the fluid through the flow channel.

In some embodiment of the invention, the hollow flexible body is having at last one outwards from the flow channel bulging area, preferable upstream the waist area. The will help to increase the movability of the annular element without causing stress or strain in the material of the hollow flexible body. This may further provide an improved response of the vale depending on the positing of the waist area

Further embodiments of the invention are defined in the dependent claims, wherein features for the second and subsequent aspects of the invention are as for the first aspect mutatis mutandis.

An advantage of the invented valve is to facilitate the positioning of a valve between two non-flexible in and outlet channels to perform opening and closing movements of the valve without causing strain on the material an to avoid strains on the mountings of the non-flexible in- and outlet channels. Strain or stress could cause lasting deformation or wear. For medical use wear such as cracks in the conduit could be an issue since it may increase the risk for contamination. Deformation of wear may also have affect the reliability of the equipment e.g. could break down in critical situations. Such drawbacks are effectively avoided by the invented valve mechanism.

Further advantages is that construction materials of the hollow flexible body and the valve seats may be autoclavable and/or the construction material in the hollow flexible body and the valve seats are disposable, or the design may comprise parts being autoclavable combined with parts being disposable. Examples of such autoclavable materials include silicone rubber, stainless steel etc.

Even further, an advantage of the invention is the simplicity in dissembling and assembling the different portions which could be crucial in medical applications. Further advantageous is that parts being hard to clean, and/or expensive to manufacture, e.g. the actuator unit, is kept separate from the flow channel with a small possibility of being contaminated.

Another advantage of the invention is that the flow channels may thus be provided with low flow resistance and low pressure drop due to its aerodynamic shape and low flow turbulence. This is particularly important in medical ventilator expiratory valve applications, as expiration normally is performed passive by the elasticity of the patient's chest and work of breathing is to be reduced.

Some embodiments of the invention, an advantage is the highly controllable collapse that can be conducted without compressing the material of the hollow flexible body when choking and/or closing the valve. Further, advantages of this embodiment are the improved sealable effect when completely abutting the valve seat.

It should be emphasized that the term "comprises/comprising" when used in this specification is taken to specify the presence of stated features, integers, steps or components but does not preclude the presence or addition of one or more other features, integers, steps, components or groups thereof.

### BRIEF DESCRIPTION OF THE DRAWINGS

These and other aspects, features and advantages of which embodiments of the invention are capable of will be apparent and elucidated from the following description of embodiments of the present invention, reference being made to the accompanying drawings, in which
Fig. 1A to C are showing an exemplary embodiment according to the principle of the invented valve mechanism;
Fig. 2A and B are showing another exemplary embodiment according to the invented valve mechanism;
Fig. 3A and B are showing a further exemplary embodiment according to the invented valve mechanism;
Fig. 4A and B are showing an exemplary embodiment according to the invented valve mechanism;
Fig. 5A to C are showing an exemplary embodiment of the invention according to the invented valve mechanism;
Fig. 6A and B are showing a exemplary embodiment of the invented valve mechanism;
Fig. 7A and E are showing exemplary embodiments of a waist configuration with diamond shapes according to the invented valve mechanism;
Fig. 8 is showing an exemplary embodiment of the invented valve mechanism with a waist having diamond shaped zones.

### DESCRIPTION OF THE PREFERRED EMBODIMENTS

Specific embodiments of the invention now will be described with reference to the accompanying drawings. This invention may, however, be embodied in many different forms and should not be construed as limited to the embodiments set forth herein; rather, these embodiments are provided so that this disclosure will be thorough and complete, and will fully convey the scope of the invention to those skilled in the art. The terminology used in the detailed description of the embodiments illustrated in the accompanying drawings is not intended to be limiting of the invention. In the drawings, like numbers refer to like elements.

The following description focuses on an embodiment of the present invention applicable to a valve to control a flow of a fluid through a flow channel and in particular to an expiratory valve. However, it will be appreciated that the invention is not limited to this application but may be applied to many other mechanical valves to control a flow.

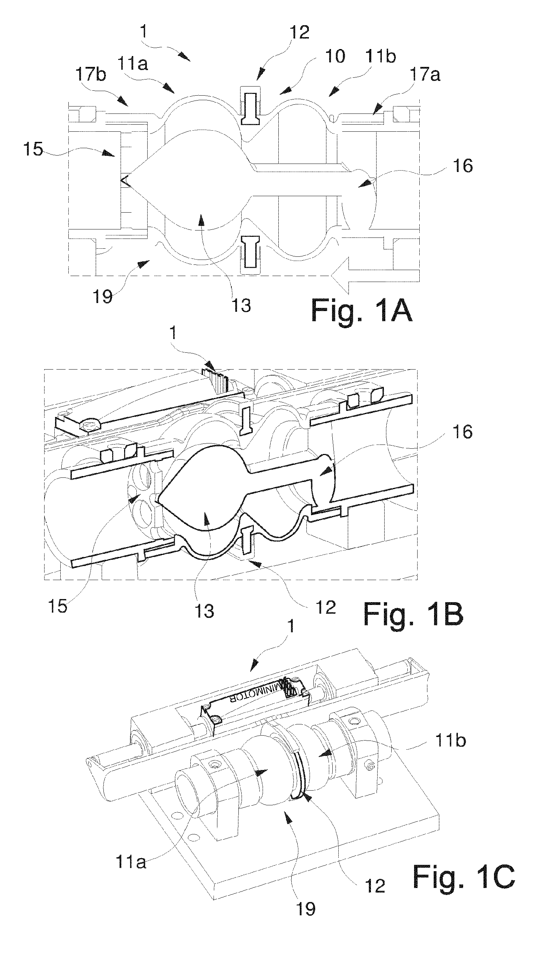

In an embodiment of the invention according to Fig. 1A to C, a valve 1 made of a hollow flexible body 19. Fig. 1A shows a cross-sectional view of the valve 1.

An advantageous design of a valve 1 according to one embodiment of the invention is to make the hollow flexible body 19 as a soft conduit made of materials such as silicone.

The flexible body 19 has in this illustrated embodiment two outwards bulging areas 11a and 11b. Between the two outwards bulging areas is a waist area 10 located, whereby a generally dumbbell shaped profile is obtained.

The flexible hollow body 19 of the valve 1 is fastened at two fixed endpoints 17a and 17b.

Inside the hollow flexible body 19 is a flow channel arranged having a centrally positioned valve seat 13. In some embodiments of the invention a valve seat 13 has a rotationally symmetric circular profile. Alternatively, in some embodiments the valve seat 13 has a rotationally symmetric conical profile. This design has the advantage of further decreasing the turbulence of the flow due to the conical shape.

The valve seat 13 is positioned and fixed using at least one strut 15, 16. Alternatively, two struts 15, 16 may be used to fix the valve seat 13 in the flow channel.

Further, an annular element 12 is arranged to circumferential at least a portion of the waist area 10. The annular element 12 may be moved in an axial direction of the flow. The axial movement of the annular element 12 may be conducted by using an actuator unit, such as a piezoelectric unit or a minimotor.

When moving the annular element 12 either towards or away from the valve seat the flow of a fluid through the flow channel may be controlled. The flow of a fluid through the flow channel is in the direction of the arrow.

The movement of the annular element 12 causes the waist area to be repositioned or reshaped. An axial movement of the annular element 12 in a direction towards the upstream part of valve seat 13 may decrease the flow through the flow channel. An axial movement of the annular element 12 in a direction away from the valve seat 13 will increase the flow through the flow channel. The increase or decrease of the flow is due to the change in the flow cross-section area of the flow channel between inner part of the waist area 10 and an upstream area of the valve seat 13. The change is caused by the repositioning and/or reshaping of the waist area 10.

The outwards, from the flow channel, bulging areas 11a, 11b may provide for the axial movement of the annular element 12 by an enhanced flexibility of the hollow flexible body 19. Thus the repositioning and/or reshaping of the waist area 10 may be conducted without causing strain or stress which may lead to lasting deformation or wear. For medical use wear such as cracks in the conduit could be an issue since it may increase the risk for contamination. Deformation of wear may also have affect the reliability of the equipment e.g. could break down in critical situations. Such drawbacks are effectively avoided by the invented valve mechanism.

In this illustrated exemplary embodiment, the valve 1 is closed by having the waist area 10 abutting an upstream area of the valve seat 13. An advantage of having the closing taking place at an upstream location is that the force of the flow will help close the valve 1, thus a faster closing response may be provided. Further, due to the force of the flow, the sealing capability of the embodiment may be increased.

Further advantages id the easy control of the valve and the force is handable.

Fig 1B illustrates the cross-section of the valve 1 from another angle. In this fig. an exemplary design of the struts 15, 16 for holding the valve seat 13 in its position in the flow channel is illustrated. Fig 1C, show an illustration of a valve 1 from the outside. Here the annular element 12 and it position at the hollow flexible body 12 and the outwards bulging areas, 11a, 11b area illustrated. A minimotor connected to conduct the movement of the annular element 12 is also illustrated.

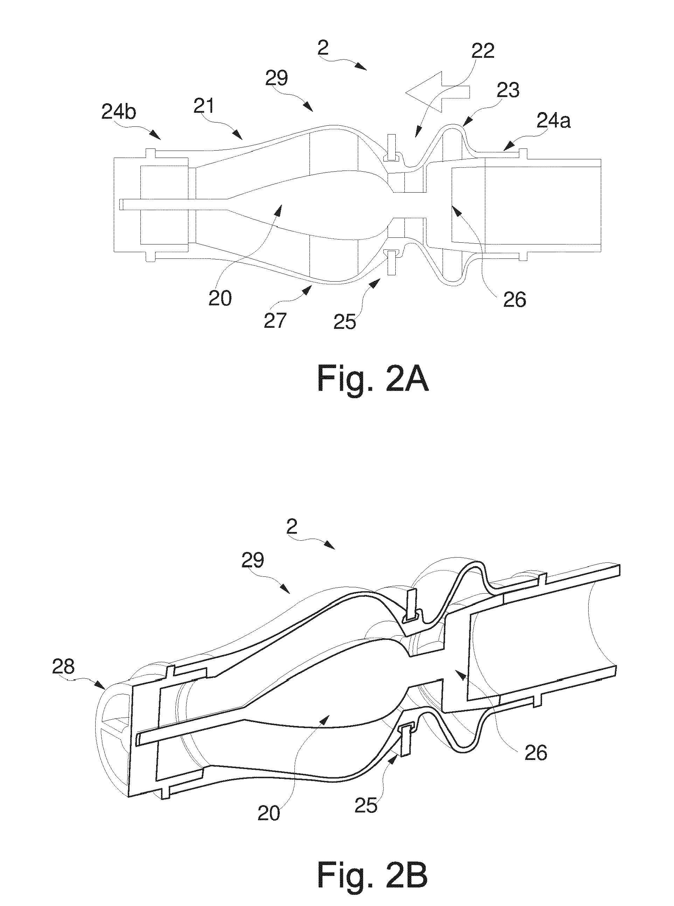

Fig. 2A and B illustrates another exemplary embodiment of a valve 2, The valve is constructed using a hollow flexible body 29 having a waist area 22. The hollow flexible body 29 is fastened at to fixed endpoints 24a, 24b. An axially movable annular element 25 is arranged within a portion of the waist area 22. Additionally and or alternatively, in some embodiments an outwards bulging area 23 may be provided to increase the repositioning and or reshaping of the waist area.

The flow through the flow channel of the illustrated valve 2 is controlled by axially moving the annular element 25 in the flow direction. The valve 2 is closed by having the waist area 2 abutting an upstream area of a valve seat 20.

Portions of the wall 21 of the hollow flexible body 29 are gradually more rigid towards one of the fixed endpoints 24b. Preferably, the gradual rigidity is obtained by gradually making the wall thicker. This will help to keep the walls uncollapsible while still providing flexibility in a point 27. Thus the repositioning and/or reshaping of the waist area 22 may be done without causing strain or stress in the material of the walls.

Additionally and/or alternatively, in some embodiments of the invention a tubular element 26 may be provided that extend into the flow channel of the valve. By having an end surface of the tubular element 26 abutting a part of the bulging area 23 when the vale is in an open position flow into the bulging area 23 is avoided. Thus, less turbulence in the flow may be provided.

In some embodiments, the tubular element 26 may have a strut in one end to hold the valve seat 20. A further strut 28 may be provided downstream of the valve seat 20.

Additionally a shoulder may be provided at an area of the valve seat 20. The shoulder is configured to accommodate an abutting area of the waist area 22 when abutting the valve seat 20 when the valve is in a closed position. Hence this configuration may improve the sealability of the closed valve.

Some advantages with this design of the invented valve is an increases aerodynamic shape providing a low pressure drop. The design also provides for a handable force.

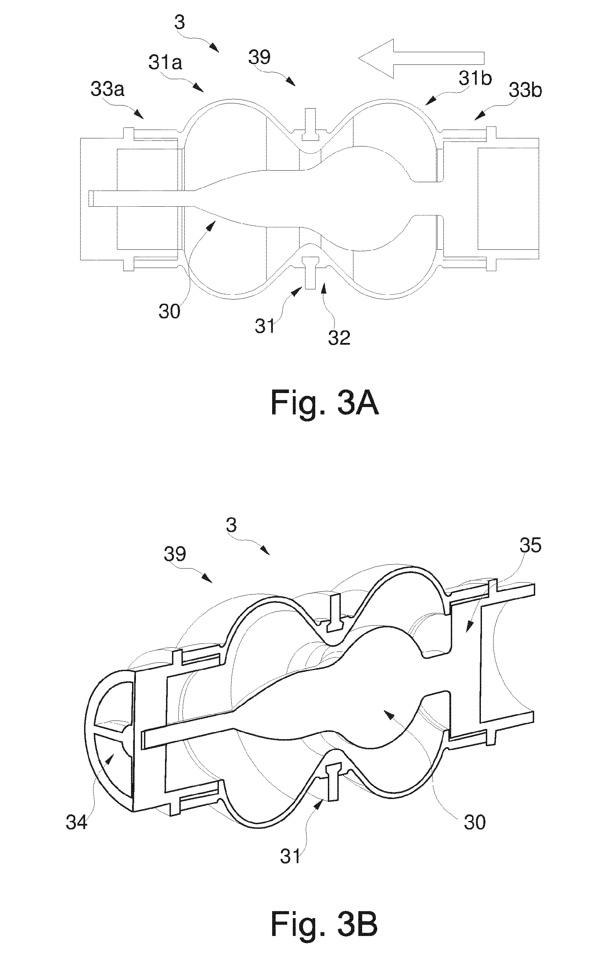

Fig. 3A and B illustrates and embodiment of a valve 3 according to the invention. The valve mechanism is similar to the valve illustrated in fig. 1A to C. The valve is constructed using a generally dumbbell shaped hollow flexible body 39 having a waist area 32 and two outwards bulging areas 31a, 31b. The hollow flexible body 39 is fastened at to fixed endpoints 33a, 33b.

The bulging areas are provided to enhance the repositioning and/or reshaping of the waist area 32 when moving an annular element 31 in an axial direction of the flow through the flow channel.

In this embodiment the valve seat 30 has a pear like shape and the closing of the valve is made by moving the annular element 31 so that the repositioned and/or reshaped waist area 32 is abutting a downstream area of the valve seat 30.

This way of closing the valve may improve the response of the valve when opening since the force of the flow will move in the same direction as the annular element 31.

The pear-shaped valve seat 30 may be hold in a position at the center of the flow channel by at least one strut 35 or 34.

Some advantages with this configuration of valve 3 is an improvement in the aerodynamic shape leading to a lower pressure drop and a handable force.

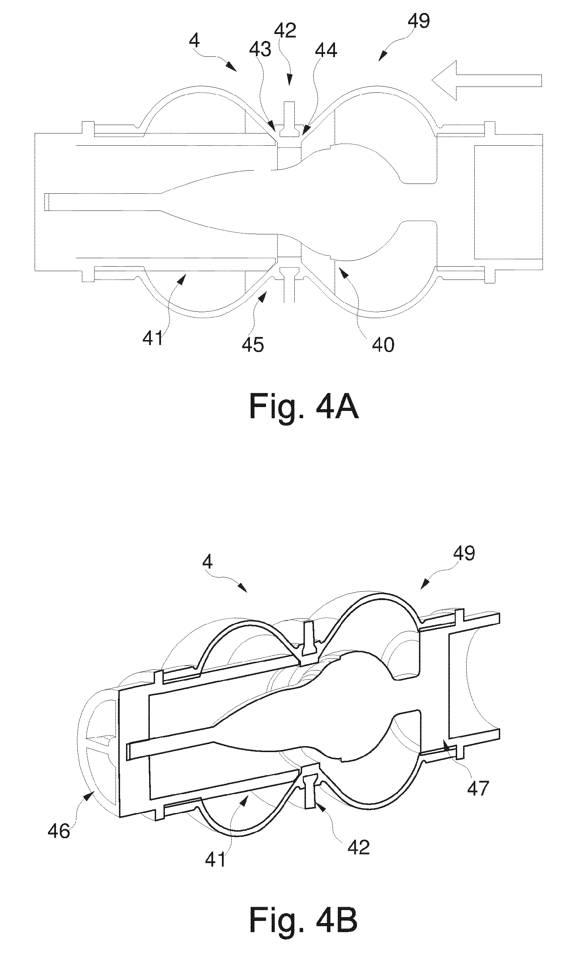

Fig. 4A and B illustrates a further embodiment of the invented valve 4. The valve works similar to the exemplary embodiments of fig. 1A to C and fig. 3A and B. In this embodiment a tubular element 41, such as a symmetrical silicone tube is extended into the flow channel of a hollow flexible body 49. By having the waist area 42 sealingly abutting an end surface 43 of the tubular element 41 when the valve 4 is in an open position fluid is prevented to enter the bulging area. Thus less turbulence in the flow may be provided since the bulging area is sealingly covered from a flow of a fluid.

The valve seat 40 is provided with a shoulder to accommodate an abutting area 44 of the waist area 42 when repositioned and/or reshaped by an annular element 45, circumferential positioned around a portion of the waist area 41, to a closed position.

The valve seat 40 may be held in a central position of the flow channel by at least one strut 46, 47.

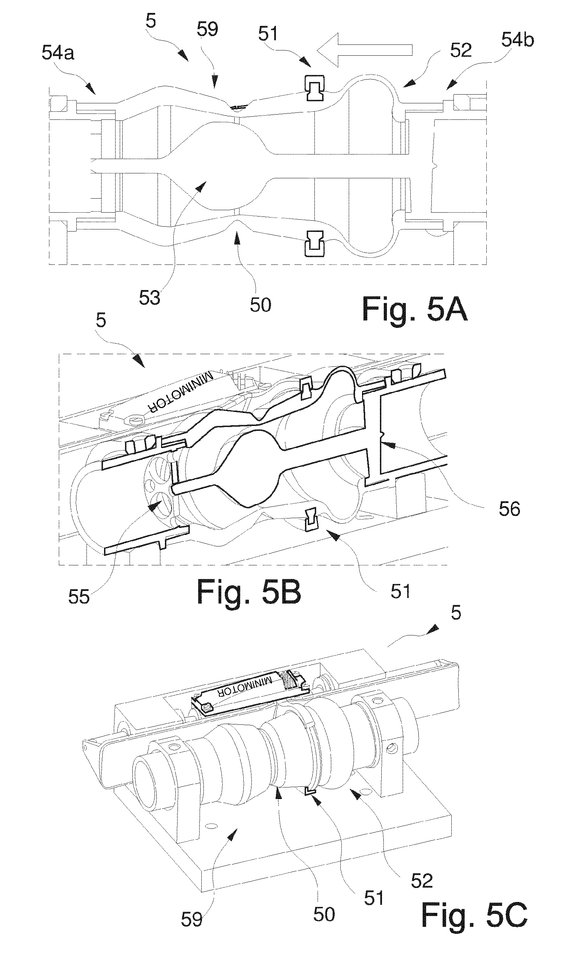

Fig. 5A to C illustrates a further exemplary embodiment of the invention.

In this embodiment the waist area 50 of the hollow flexible body 59 is configured to controllably collapse against at valve seat 53 by an axial movement of an annular element 51. The annular element 51 is circumferential positioned between the waist area 50 and at least one of the fixed endpoints 54a, 54b. Preferably between the waist area 50 and the fixed endpoint 54b to have a smooth inner surface downstream a valve seat 53.

Additionally, in some embodiments of the invention a outwards bulging area 52 may be provided to enhance the axial movement of the annular element 51 without casing any stress or strain in the material of the hollow flexible body 59.

The valve seat 53 may have any shape previously described. The valve seat 53 may be hold in its centre position of the flow channel by at least one strut 55, 56.

Some advantages with this embodiment is the high aerodynamic shape providing low turbulence and low pressure drop.

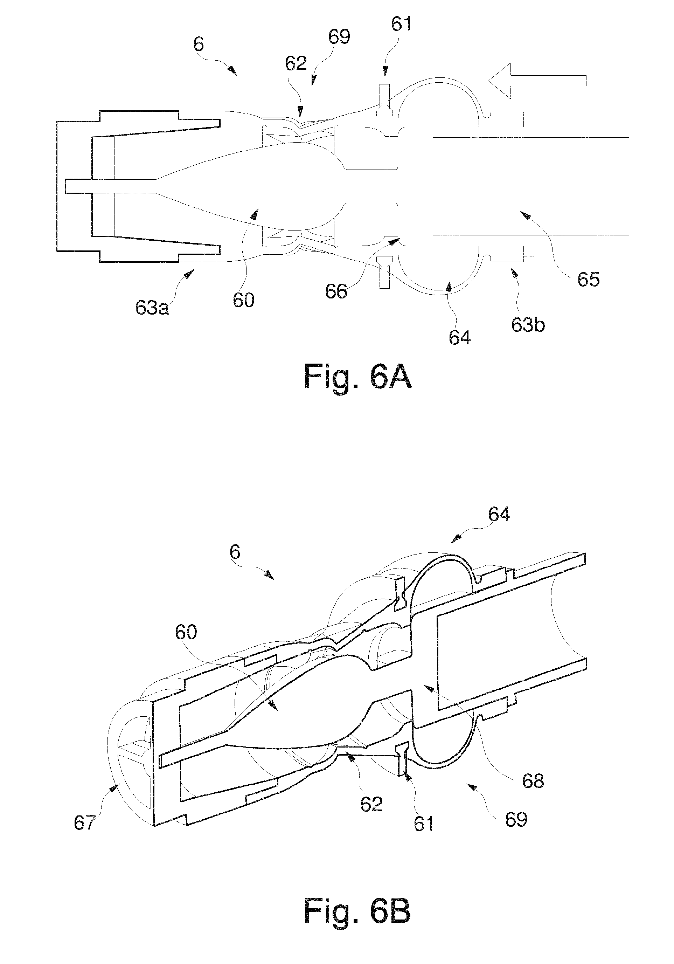

Fig. 6A and B illustrates an additional exemplary embodiment of a valve 6 similar to the valve in fig. 5A and B. In this embodiment a tubular element 65 is positioned into the flow channel of the hollow flexible body 69. The tubular element has similar advantages as the tubular element in fig. 2A and B. Here, an end surface 66 of the tubular element 65 abuts an area of the inner surface of the hollow flexible body when the valve is in an open position. Thus fluid is prevented to enter the bulge area 64. The inner surface is held in an abutting positing by an annular element 61.

By moving the annular element axially in a direction of the flow the flow through the flow channel may be controlled by controllably collapsing the waist area 62 towards the valve seat 60.

The valve seat 60 is held in place by at least one strut 67, 68. And the hollow flexible body 69 is fastened at the fixed endpoints 63a, 63b.

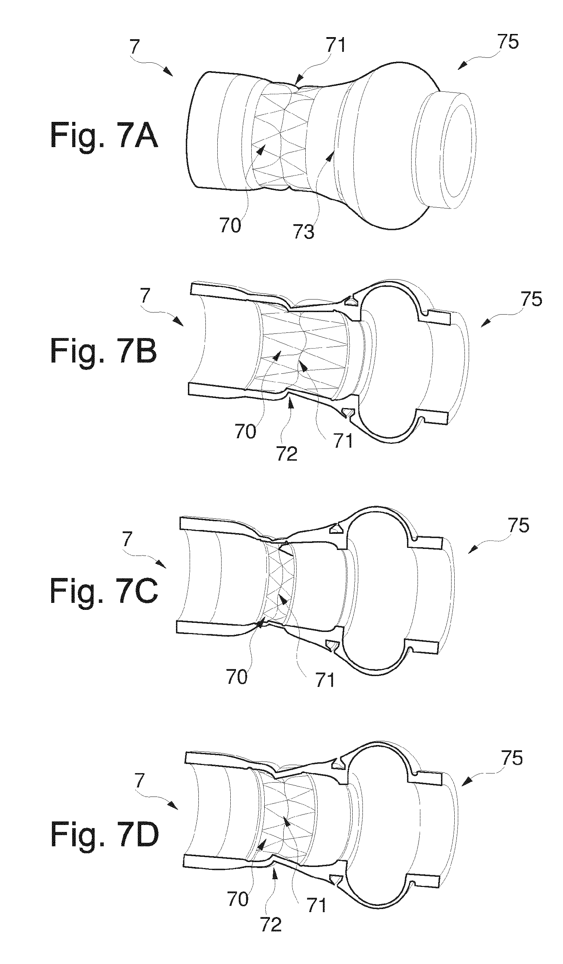

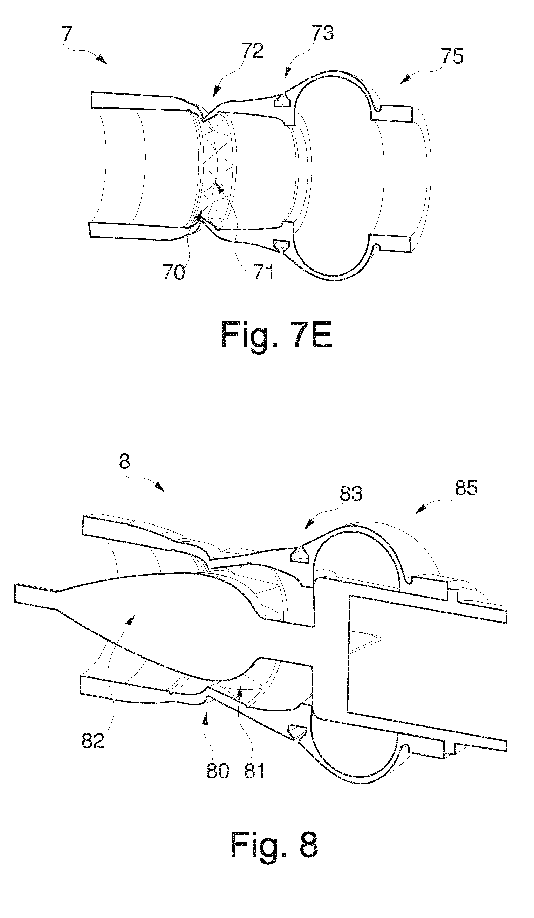

Fig 7A to E illustrates a hollow flexible body 7 of a valve. The hollow flexible body 7 has a preferred and advantageous configuration of a hollow collapsible waist area 71. The waste area 71 has diamond shaped zones 70 along the circumference of the waist area 72 with a notch 72 in the middle. For illustrating pulpous the position of an annular element 73 and a bulge area 75 is shown.

When collapsed there will be no great stress or strain in the material of the hollow flexible body since the material will be positioned twinedly along the inner diameter of the waist area, such as in a sinusoid. While lowering the stress and strain of the material, this will further increase the sealability and the control of the collapse. The stress and train will be lowered since there will be no compression of the material when collapsed according to this principle. Further, this will prove an improvement of the control of a flow through the valve.

The different fig 7A to E shows different shapes, shapes and angles that may be used for different kind of configurations of a valve. The configuration used may depend on reposes needed, sealability, the material and the dimensions of the valve and flow channel.

Fig 8. Illustrates an exemplary embodiment similar to the embodiment in fig 6a and B but here having a collapsible waist area 80 with diamonds shaped zones 81, according to the principle of the waist area previously described for fig 7A to E.

Alternatively the principle of the configuration of a waist area according to fig. 7A to E may be used in conjunction with any embodiment described herein.

Further, the embodiments according to the invention may preferably be constructed using materials of the hollow flexible body and the valve seats being autoclavable and/or the construction material in the hollow flexible material and the valve seats are disposable, or the design may comprise parts being autoclavable combined with parts being disposable. Examples of such autoclavable materials include silicone rubber, stainless steel etc.

A main principle of the invention is to improvingly facilitate the positioning of the valve between two non-flexible in and outlet channels, additional bulging areas may be utilized so that the axial movement of the annular element repositioning and/or reshaping a waist area can be carried out. Thus strain on the material or on the mountings of the non-flexible in- and outlet channels is avoided. Strain or stress could cause lasting deformation or wear. For medical use wear such as cracks in the conduit could be an issue since it may increase the risk for contamination. Deformation of wear may also have affect the reliability of the equipment e.g. could break down in critical situations. Such drawbacks are effectively avoided by embodiments.

Further the invention provides for an improved pressure drop and less turbulence of the flow by providing an improved aerodynamic shape for the flow through the valve. Further the principles of the invention provides for an increased controllability of the flow through the flow channel.

The principle of the function of the different parts of the described device may be regarded as the principle and steps for a method to mechanically control a flow in a flow channel.

As will be appreciated by one of skill in the art, the present invention may be embodied as device, system or method.

While several embodiments of the present invention have been described and illustrated herein, those of ordinary skill in the art will readily envision a variety of other means and/or structures for performing the functions and/or obtaining the results and/or one or more of the advantages described herein, and each of such variations and/or modifications is deemed to be within the scope of the present invention. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings of the present invention is/are used.

## Claims

1. A valve comprising:
- a hollow flexible body having a waist area positioned between two fixed end points;
- a flow channel located inside said hollow flexible body for passage of a fluid;
- a valve seat centrally positioned in said flow channel;
- an annular element circumferentially arranged around said hollow flexible body;
wherein,
said annular element is arranged to be movable towards or away from said valve seat in an axial direction along said flow channel by an actuator unit, and whereby said waist area is at least partially repositioned and/or reshaped to control a flow of said fluid through said flow channel.

2. The valve according to claim 1, wherein said hollow flexible body having at last one outwards from said flow channel bulging area, preferable upstream said waist area.

3. The valve according to any of claims 1 to 2, wherein said hollow flexible body having walls that are gradually more rigid towards at least one of said two fixed end points.

4. The valve according to any of claims 1 to 3,
wherein said hollow flexible body having walls that are gradually thicker towards at least one of said two fixed end points.

5. The valve according to any of claims 1 to 4, wherein said waist area has an abutting area configured to after reposition and/or deformation of said waist area sealingly abut said valve seat.

6. The valve according to any of claims 1 to 5, wherein said annular element is circumferentially arranged around a portion of said waist area.

7. The valve according to claim 6, wherein an abutting area of said waist area is arranged to sealingly abut said valve seat from upstream upon repositioning and/or reshaping said waist area.

8. The valve according to claim 6, wherein an abutting area of said waist area is arranged to sealingly abut said valve seat from downstream upstream repositioning and/or reshaping said waist area.

9. The valve according to claim 8, wherein said valve seat is pear shaped.

10. The valve according to any of claims 7 to 9, wherein said valve seat has a shoulder to accommodate said abutting area to close said valve.

11. The valve according to any of claims 1 to 5, wherein said annular element is circumferentially arranged around said hollow flexible body and between said waist area and one of said two fixed end points.

12. The valve according to any of claim 1 to 11, wherein said waist area is configured to be in an axially collapsed state to sealingly abut said valve seat and/or to be in an uncollapsed state, upon axial movement of said annular element.

13. The valve according to claim 12 wherein said waist area has diamond shaped zones with a central notch.

14. The valve according to claim 13, wherein said notch of said diamond shaped zones is configured to provide a twining shape along the inner diameter of the waist when in said collapsed state, preferably sinusoidal.

15. The valve according to any of claims 2 to 14, wherein a tubular element is extending into said flow channel downstream and/or upstream, and is having an end surface arranged to abut an inner wall of said hollow flexible body or to abut said waist area in an open state of said valve.
